# EUROPEAN PATENT SPECIFICATION

(11) **EP 1 992 383 B1**
(45) Date of publication and mention of the grant of the patent: **22.04.2020**
(21) Application number: 07715234.6
(22) Date of filing: 06.03.2007
(51) Int. Cl.: A61M 25/01

(54) **GUIDE WIRE**
FÜHRUNGSDRAHT
FIL GUIDE

(30) Priority: 06.03.2006 JP 2006059902; 28.04.2006 JP 2006125809
(43) Date of publication of application: 19.11.2008
(73) Proprietor: Terumo Kabushiki Kaisha, Tokyo 151-0072 (JP)
(72) Inventor: TAMAI, Noriyuki, Fujinomiyashi, Shizuoka 418-0015 (JP); SHIRAKAWA, Katsuhiro, Fujinomiyashi, Shizuoka 418-0015 (JP); YAGI, Hiroshi, Fujinomiyashi, Shizuoka 418-0015 (JP)
(74) Representative: Dossmann, Gérard
(86) International application number: PCT/JP2007/054266
(87) International publication number: WO 2007/105531

(56) References cited:
- EP-A- 0 778 044
- WO-A-96/19148
- WO-A-98/37923
- WO-A-99/23958
- WO-A-99/52421
- WO-A-2004/110519
- JP-A- 11 076 415
- JP-A- 11 076 415
- JP-A- 2004 154 286
- US-A- 3 757 768
- US-A1- 2005 113 862
- US-A1- 2005 228 418
- US-B1- 6 524 301

## Description

### TECHNICAL FIELD

The present invention relates to a guide wire used for introducing a medical device of a catheter, an introducer kit or the like, which is used for aiming a medical treatment or a diagnosis, until a desired region in a blood vessel.

### BACKGROUND ART

The guide wire is used when introducing and indwelling a medical device of a catheter, an introducer kit or the like in a blood vessel on an occasion of executing a diagnosis and a medical treatment of the blood vessel percutaneously. It was a mainstream in the past that a region when introducing a medical device of a catheter or the like into a blood vessel was selected to be a femoral (FEMORAL), but in recent years, the region thereof is shifting to a brachial (BRACHIAL) and, in particular, to a radial (RADIAL) in order to lighten the burden with respect to the patient, and there has been desired a guide wire including, for example, a J-shape at the distal tip thereof which can be used safely in a blood vessel of an arm portion that often includes branches and meanders and also, which is excellent in steerability.

In the past, when a guide wire including a J-shape at the distal tip thereof was inserted into an introducing needle, a catheter or the like, there has been used a supplemental tool (inserter) for making the insertion easier, but in a case, in particular, in which the curvature radius of the curve portion of the J-shape is small, an operation for inserting a guide wire once again into the inserter when exchanging the catheter or the like was complicated.

In order to solve the above-mentioned problem, there is disclosed a tool in which a supplemental tool is made to be unnecessary when inserting a wire into an introducing needle, a catheter, a sheath or the like by designing the angle formed by the extended line in the direction of the distal tip linear portion and the wire base line to be 40 to 70° with respect to the distal shape of the guide wire (for example, see Patent Document 1).

With respect to the distal shape of the guide wire, there is disclosed a guide wire preformed in a multi-bending shape for making it difficult to enter a side opening of a tube for medical treatment (for example, see Patent Document 2). Also, there is disclosed a guide wire which includes two curve portions facing different directions in order to control the direction of the distal tip of the guide wire (for example, see Patent Document 3).

In case of a guide wire whose distal tip is formed to be in a J-shape as seen in Patent Document 1, it is necessary to stretch the J-shaped distal portion once and thereafter to insert it into an introducing needle, catheter or a sheath, so that there often happened a case in which steerability is bad.

Also, with respect to the guide wire of the Patent Document 2, the inner diameter of the tube for medical treatment to be used is known beforehand, so that in case of using a guide wire having a shape corresponding to the inner diameter thereof, it was difficult for the guide wire to come out from the side opening of the tube for medical treatment. However, the blood vessel is different in the diameter thereof depending on the individual organism or the region, so that in case of using the guide wire of the Patent Document 2 for the blood vessel, it was necessary to separately use a guide wire which has a different shape for every patient or for every region so as not to erroneously-enter into a side-branch of a blood vessel. It is difficult also for the guide wire of the Patent Document 3 to erroneously-enter into a side-branch in case of a blood vessel having a thin side-branch, but in case of a blood vessel having a thick side-branch, it happens that the guide wire may erroneously-enter into the side-branch and it became difficult to reach the aimed region and therefore, the steerability was bad.

Also, there is a guide wire in guide wires of Patent Document 4 in which the shape thereof is formed to be such a shape that it is possible to select branches of both the vascular channels having large and small diameters, but it is easy to erroneously-enter into a branch for that reason and it happens that it will spend time for the operation after all in case of introducing it from the radial as mentioned above.

Further, a guide wire of Patent Document 5 is a guide wire having an S-shape for the distal shape thereof in order to introduce it into a blood vessel branch, but the guide wire having an S-shape distal tip faces the hand direction at the most distal tip thereof, so that it is difficult to insert it into an introducing needle or a catheter and an inserter must be used every time.

[Patent Document 1] Japanese unexamined patent publication No. 2004-181184
[Patent Document 2] Japanese unexamined patent publication H11-76415
[Patent Document 3] Japanese unexamined PCT patent publication No. 2003-530132
[Patent Document 4] Japanese unexamined PCT patent publication No. 2003-508168
[Patent Document 5] Japanese utility model patent publication S61-7736
US Patent Document US 2005/0113862 A1 discloses a control element, such as a guidewire, connected to a surrounding sheath and an elastic bias section.

### DISCLOSURE OF THE INVENTION

An object of the present invention is to propose a guide wire excellent in steerability.

In order to achieve the above-mentioned object, a guide wire of the present invention includes the features of claim 1.

According to the present invention like this, the steerability thereof becomes excellent when operating the guide wire. In particular, as a guide wire for introduction from a radial-artery which is introduced from a radial artery of a wrist, it is difficult to erroneously-enter into side-branches with respect to inter-individual differences and various blood vessel diameters reaching until the left ventricle and it is possible to insert it smoothly until the aimed region.

Also, with respect to a guide wire of the present invention, it is preferable for the most distal tip of aforesaid guide wire to be positioned between the line contacting with both aforesaid first curve portion and aforesaid third curve portion and a line which is in parallel with aforesaid line and contacts with aforesaid second curve portion.

Thus, when inserting a guide wire into a tubular medical device such, for example, as an introducing needle, a catheter, a sheath and the like, it is possible to execute the insertion operation thereof easily and also certainly.

Also, with respect to a guide wire of the present invention, the direction of the portion shifting from aforesaid first curve portion to aforesaid second curve portion has a larger angle with respect to aforesaid axis line compared with the direction which aforesaid most distal tip is directed with respect thereto.

Thus, when inserting a guide wire into a tubular medical device such, for example, as an introducing needle, a catheter, a sheath and the like, it is possible to execute the insertion operation thereof easily and also certainly.

Also, with respect to a guide wire of the present invention, it is preferable for the portion shifting from aforesaid second curve portion to aforesaid third curve portion to be approximately in parallel with respect to aforesaid axis line.

Thus, when inserting a guide wire into a tubular medical device such, for example, as an introducing needle, a catheter, a sheath and the like, it is possible to execute the insertion operation thereof easily and also certainly.

Also, with respect to a guide wire of the present invention, it is preferable for the portion shifting from aforesaid second curve portion to aforesaid third curve portion to be in a direction spreading with respect to aforesaid axis line.

Thus, when inserting a guide wire into a tubular medical device such, for example, as an introducing needle, a catheter, a sheath and the like, it is possible to execute the insertion operation thereof easily and also certainly.

Also, with respect to a guide wire of the present invention, it is preferable for the portion shifting from aforesaid second curve portion to aforesaid third curve portion to be in a direction being narrowed with respect to aforesaid axis line.

Thus, when inserting a guide wire into a tubular medical device such, for example, as an introducing needle, a catheter, a sheath and the like, it is possible to execute the insertion operation thereof easily and also certainly.

Also, in order to achieve the above-mentioned object, a guide wire of the present invention includes a distal portion and a main body portion, and the guide wire is characterized in that aforesaid distal portion is provided with a curve portion.

According to the present invention like this, the steerability thereof becomes excellent when operating the guide wire.

Also, with respect to a guide wire of the present invention, aforesaid first curve portion has higher flexibility compared with aforesaid main body portion and aforesaid second curve portion has higher flexibility compared with aforesaid first curve portion.

Thus, in case of inserting a guide wire into a curved blood vessel, it is possible for the distal portion of the guide wire to follow the shape of the blood vessel thereof. Consequently, the steerability when operating a guide wire is improved. Even if the most distal tip of the guide wire gets into a branch, it turns around, advances in the main blood vessel and does not erroneously-enter into a branch.

Also, in order to achieve the above-mentioned object, a guide wire of the present disclosure includes a distal portion and a main body portion, and the guide wire is characterized by: a first curve portion provided at aforesaid distal portion; a second curve portion included on the distal side of aforesaid first curve portion and curved to the opposite direction with respect to aforesaid first curve portion; and a third curve portion included on the distal side of aforesaid second curve portion and curved to the opposite direction with respect to aforesaid second curve portion, wherein aforesaid first curve portion has higher flexibility compared with aforesaid main body portion, and aforesaid second curve portion has higher flexibility compared with aforesaid first curve portion.

According to the present disclosure like this, the steerability thereof becomes excellent when operating the guide wire. Even if the most distal tip of the guide wire gets into a branch, it turns around, advances in the main blood vessel and does not erroneously-enter into a branch.

Also, with respect to a guide wire of the present disclosure, it is preferable for aforesaid third curve portion to have higher flexibility compared with aforesaid second curve portion or the same flexibility compared therewith.

Thus, in case of inserting a guide wire into a curved blood vessel, it is possible for the distal portion of the guide wire to follow the shape of the blood vessel thereof. Consequently, the steerability when operating a guide wire is improved.

Also, with respect to a guide wire of the present disclosure, it is preferable for aforesaid guide wire to be composed of a core wire including a distal portion and a coating portion which covers at least the distal portion of aforesaid core wire and which is constituted by a resin.

Thus, the steerability thereof becomes more excellent when operating the guide wire.

Also, with respect to a guide wire of the present disclosure, it is preferable for the distal portion of aforesaid core wire includes a flat portion.

Thus, higher flexibility is exhibited at the flat portion.

Also, with respect to a guide wire of the present disclosure, it is preferable for the core wire at which aforesaid third curve portion is positioned to be a flat portion and for aforesaid third curve portion to have higher flexibility compared with aforesaid second curve portion.

Thus, much higher flexibility is exhibited at the flat portion and consequently, the steerability thereof becomes more excellent when operating the guide wire.

### BRIEF DESCRIPTION OF DRAWINGS

FIG. 1 is a partially enlarged view showing one exemplified embodiment of a guide wire of the present invention;
FIG. 2 is a partially enlarged view showing another exemplified embodiment of a guide wire of the present invention;
FIG. 3 is a perspective view showing an internal structure of a guide wire of the present invention;
FIG. 4 is a perspective view showing a portion of an internal structure of a guide wire of the present invention;
FIG. 5 is a partially enlarged view showing another exemplified embodiment of a guide wire of the present invention;
FIG. 6 is an end view showing another exemplified embodiment of a guide wire of the present invention;
FIG. 7 is an end view showing another exemplified embodiment of a guide wire of the present invention;
FIG. 8 is an end view showing another exemplified embodiment of a guide wire of the present invention;
FIG. 9 is an end view showing another exemplified embodiment of a guide wire of the present invention;
FIG. 10 is an end view showing a guide wire according to another exemplified embodiment of the present invention;
FIG. 11 is a view (plan view) showing a specific configuration of the guide wire shown in FIG. 1;
FIG. 12 is a view (plan view) showing a specific configuration of a guide wire of a comparative example 1;
FIG. 13 is a view (plan view) showing a specific configuration of a guide wire of a comparative example 2;
FIG. 14 is a view (plan view) showing a specific configuration of a guide wire of a comparative example 3;
FIG. 15 is a view (plan view) showing a specific configuration of a guide wire of a comparative example 4;
FIG. 16 is a view (plan view) showing a specific configuration of a guide wire of a comparative example 5;
FIG. 17 is a view showing an evaluation tool used when respective guide wires shown in FIG. 11 to FIG. 16 are evaluated respectively; and
FIG. 18 is a view showing an evaluation tool used when respective guide wires shown in FIG. 11 to FIG. 16 are evaluated respectively.

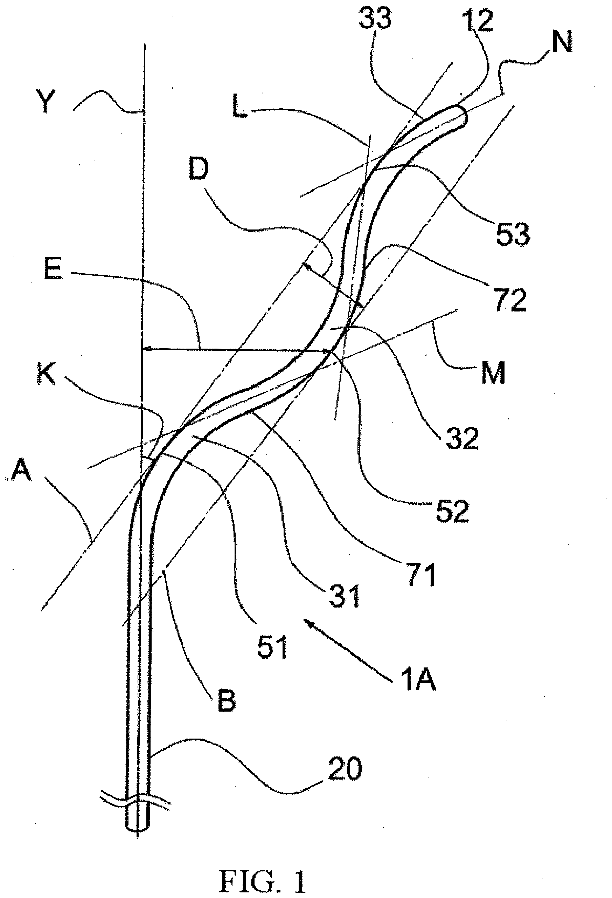

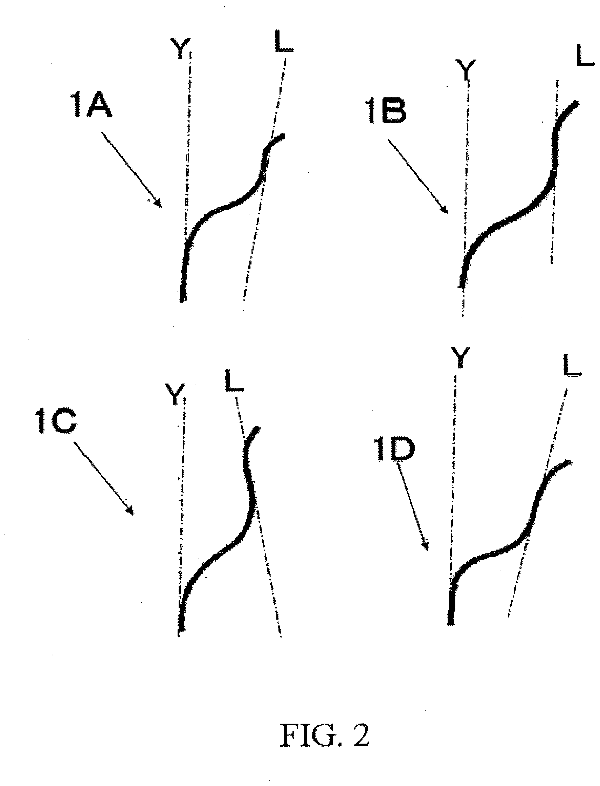

FIG. 1 is a partially enlarged view showing one exemplified embodiment of a guide wire of the present invention and FIG. 2 is a partially enlarged view showing other exemplified embodiments of the guide wire of the present invention. Hereinafter, the present invention will be explained based on a guide wire 1A which is one exemplified embodiment. As shown in FIG. 1, the guide wire 1A which is one exemplified embodiment of the present invention is a guide wire composed of a distal portion 10 and a main body portion 20. Curve portions are included at the distal portion 10. The distal portion 10 includes a first curve portion 31 subsequent to the distal side of the main body portion 20. The distal portion 10 includes a second curve portion 32 which is included on the distal side of the first curve portion 31 and is curved to the opposite direction with respect to the first curve portion 31. It is also allowed to include a linear portion between the first curve portion 31 and the second curve portion 32.

The distal portion 10 includes a third curve portion 33 which is included on the distal side of the second curve portion 32 and is curved to the opposite direction with respect to the second curve portion 32. It is also allowed to include a linear portion between the second curve portion 32 and the third curve portion 33. The distal side of the third curve portion 33 is terminated by the most distal tip 12. The first curve portion 31, the second curve portion 32 and the third curve portion 33 are constituted in the same plane.

The guide wire 1A includes a line A contacting with both the first curve portion 31 and the third curve portion 32. The line A passes a contact point 51 of the first curve portion 31 and a contact point 53 of the third curve portion 33. The line A forms an obtuse angle with respect to an axis line Y of the main body portion 20 in its natural state. Here, "natural state" means a state in which external force is not applied to the guide wire 1A. It is preferable for the angle K between the line A and the axis line Y to be 20 to 45 degrees and, more preferably, to be 25 to 42 degrees.

The most distal tip 12 of the guide wire 1A is positioned between the line A which contacts with both the first curve portion 31 and the third curve portion 32 and a line B which is in parallel with the line A and contacts with the second curve portion 32. The line B passes a contact point 52 of the second curve portion 32.

The direction M of a portion 71 shifting from the first curve portion 31 to the second curve portion 32 has a larger angle with respect to the axis line Y compared with the direction N which the most distal tip 12 faces. The direction L of a portion 72 shifting from the second curve portion 32 to the third curve portion 33 is a direction spreading toward the distal direction with respect to the axis line Y.

It is preferable for the distance D between the line A and the line B to be 2 to 11mm and, more preferably, to be 4 to 9mm. If the distance D is smaller than 2mm, the most distal tip 12 will contact with the blood vessel wall when advancing in the blood vessel and more chances occur for the erroneous-entering and if it is larger than 11mm, it happens that the most distal tip 12 will contact with the blood vessel wall in case of advancing in a comparatively thin blood vessel. It is preferable for the distance E between a vertex 52 of the second curve portion 32 and the axis line Y of the main body portion 20 to be 9 to 16mm and, more preferably, to be 10 to 15mm. If the distance E is smaller than 9mm, the second curve portion 32 does not contact with the blood vessel wall when advancing in a rather thick blood vessel and the most distal tip 12 will approach or contact with the blood vessel wall, in which more chances occur for the erroneous-entering to side-branches and if it is larger than 16mm, the second curve portion 32 becomes in a state of spreading when advancing in a rather thin blood vessel and the most distal tip 12 is reflexed, in which more chances occur for the contact with the blood vessel wall and possibility of the erroneous-entering becomes high.

The guide wire 1A includes a core wire and a coating portion which covers at least the distal portion of the core wire and which is constituted by a resin. It is preferable for the core wire to be an NiTi alloy wire. It is preferable for the resin of the coating portion to be polyurethan. It is preferable for the front face of the coating portion to be coated with hydrophilic Polymer. The core wire has a tapered shape at the distal portion 10, so that the distal portion 10 is more flexible than the main body portion 20.

It is easy for the guide wire 1A to be inserted into an introducing needle, a catheter or a sheath. Specifically, it is possible to insert the guide wire 1A into a catheter including a catheter main body of a tube shape having elasticity and a hub installed at the proximal portion of the catheter main body and into aforesaid hub from the distal side thereof easily.

Also, it is difficult for the guide wire 1A to erroneously-enter into a side-branch in a supposed blood vessel and it can reach an aimed region speedily. Specifically, when inserting the guide wire 1A into a blood vessel and in a case in which the blood vessel thereof is in a straight line shape, the most distal tip 12 of the guide wire 1A is prevented or repressed from touching the blood vessel wall. Also, in a case in which there exists, at a position of a blood vessel into which the guide wire 1A is inserted, a side-branch (blood vessel) branched from the blood vessel thereof, the distal portion 10 of the guide wire 1A is prevented from getting into the side-branch unwillingly. Thus, it is possible for the guide wire 1A to reach an aimed region in a blood vessel speedily.

FIG. 2 shows guide wires 1B, 1C and 1D as other exemplified embodiments other than the guide wire 1A mentioned above.

With respect to the guide wire 1B, the direction L of the portion shifting from the second curve portion to the third curve portion is approximately in parallel with respect to the axis line Y. Also, with respect to the guide wire 1C, the direction L of the portion shifting from the second curve portion to the third curve portion is a direction to be narrowed toward the distal tip with respect to the axis line Y. With respect to the guide wire 1D, similarly as the guide wire 1A, the direction L is a direction spreading toward the distal tip with respect to the axis line Y.

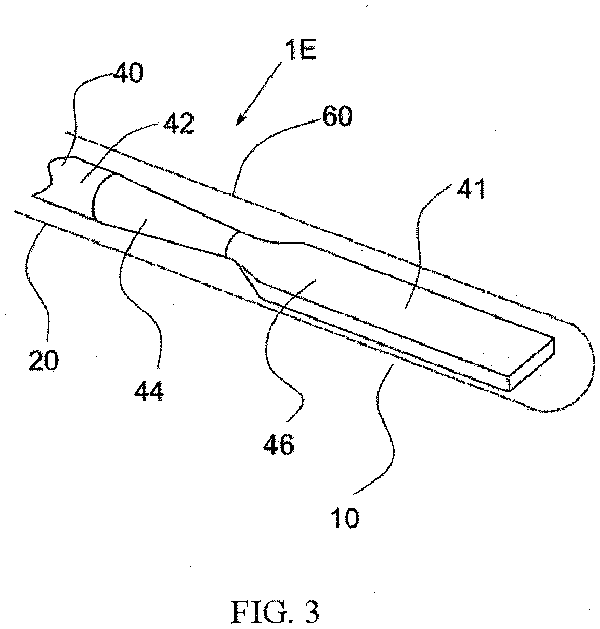

FIG. 3 is a perspective view showing an internal structure of a guide wire of the present invention. In order to show the internal structure, the curve portions of the distal portion 10 are omitted. A guide wire 1E shown in FIG. 3 is constituted by a core wire 40 including a core wire distal portion 41 and a coating portion 60 which covers at least the core wire distal portion 41 and which is composed of a resin. The core wire 40 is constituted by a core wire main body portion 42, a taper portion 44 and the core wire distal portion 41. With respect to the material of the core wire 40, NiTi alloy or stainless steel can be cited. It is preferable for the core wire 40 to be composed of a superelastic alloy. The cross-section of the core wire main body portion 42 is in a circular shape. The distal side of the core wire main body portion 42 is provided with the taper portion 44. It is constituted such that the taper portion 44 becomes smaller gradually in its outer diameter toward the distal tip. It is constituted such that the taper portion 44 of FIG. 3 becomes smaller at a constant rate in its outer diameter toward the distal tip, but it is also allowed to employ a structure in which the taper angle changes at a portion of the taper portion. For example, it is allowed to employ a structure in which the taper angle on the proximal side is larger than the taper angle on the distal side. Also, it is allowed to employ a structure in which the taper angle on the proximal side is smaller than the taper angle on the distal side. There is provided on the distal side of the taper portion 44 with the core wire distal portion 41. The core wire distal portion 41 includes a flat portion 46. The flat portion 46 has width and thickness and the width is larger than the thickness. It becomes easier for the flat portion 46 to be bent in the thickness direction. It is allowed for the core wire distal portion 41 to include an outer diameter uniform portion on the distal side of the taper portion 44. In this case, a shift portion intervenes on the distal side of the outer diameter uniform portion and the flat portion 46 follows. The cross-section area of the core wire main body portion 42 is larger than the cross-section area of the core wire distal portion 41. It is preferable for the flat portion 46 to be superelastic. In a Load-Distortion curve, it is preferable for the elastic modulus in the elastic region of the flat portion 46 to be smaller than the elastic modulus in the elastic region of the core wire main body portion 42. Based on such a constitution, it is possible to secure flexibility even if the thickness of the flat portion 46 is not made to be extremely thin.

The coating portion 60 covering the core wire distal portion 41 is composed of a resin such as polyurethane and the like. The coating portion 60 covers the core wire distal portion 41, the core wire main body portion 42 and the taper portion 44, but it is also allowed to cover only the core wire distal portion 41. In addition, it is also allowed for the coating portion 60 to cover only the core wire distal portion 41 and the taper portion 44.

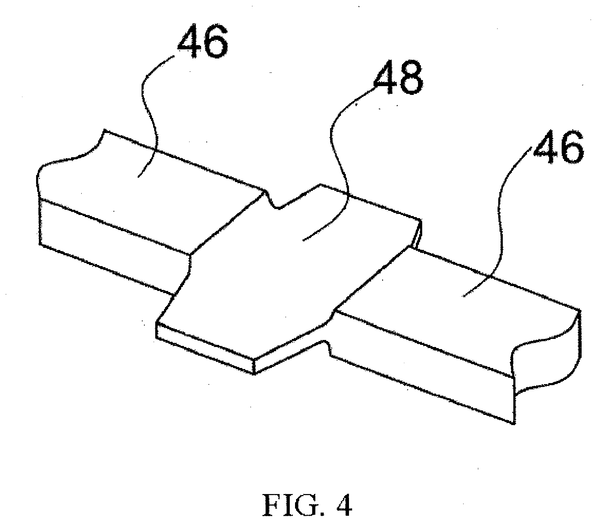

It is also allowed for the flat portion 46 of the core wire distal portion 41 to be provided with an elastic portion. One example thereof is, as shown in FIG. 4, to provide an elastic portion 48 which is made to be thinner with respect to the thickness of the flat portion 46 on the way of the flat portion 46. The width of the elastic portion 48 is constituted so as to be wider than the width of the flat portion 46. In view of an aspect that the strength of the elastic portion 48 is to be maintained, it is preferable to employ a structure in which the cross-section area of the flat portion 46 and the cross-section area of the elastic portion 48 are approximately equal. It is also allowed to employ a structure in which the width of the elastic portion 48 is made to be same as the width of the flat portion 46 by making the thickness of the elastic portion 48 thinner than the thickness of the flat portion 46. For another example of the elastic portion, it is also allowed to employ the elastic portion by changing the material property using thermal treatment or the like. In FIG. 3, the curve portions of the distal portion 10 are omitted in order to show the internal structure, but it should be noted with respect to the curve portions that the shapes of the guide wires 1A, 1B, 1C and 1D mentioned above can be applied thereto.

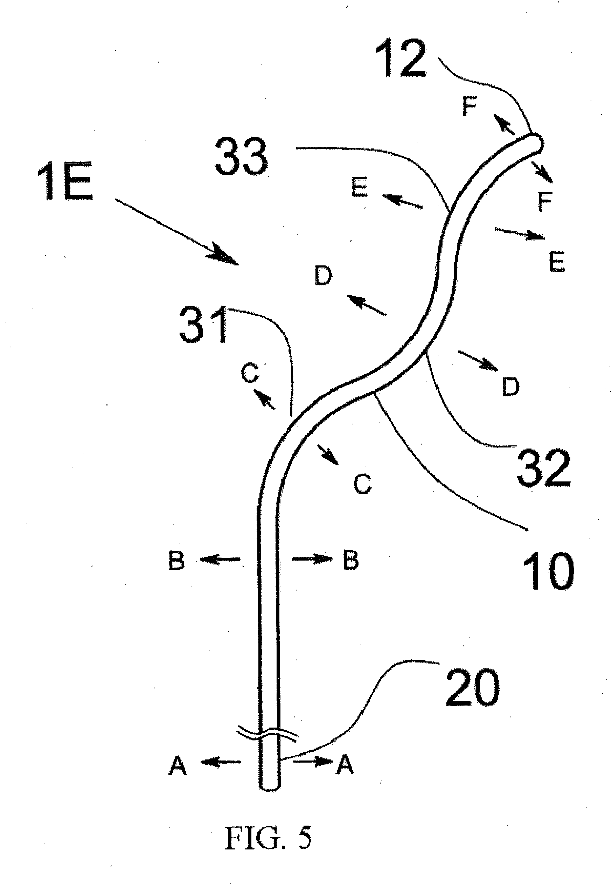

FIG. 5 is a partially enlarged view showing another exemplified embodiment of a guide wire of the present invention. A guide wire 1E provided with the structure shown in FIG. 3 in addition to the guide wire 1A having the above-mentioned shape is constituted by the distal portion 10 and the main body portion 20. The distal portion 10 is provided with the first curve portion 31, the second curve portion 32 and the third curve portion 33. The second curve portion 32 is included on the distal side of the first curve portion 31. The second curve portion 32 is curved to the opposite direction with respect to the first curve portion 31. The third curve portion 33 is included on the distal side of the second curve portion 32. The third curve portion 33 is curved to the opposite direction with respect to the second curve portion 32. The first curve portion 31 has higher flexibility compared with the main body portion 20. The second curve portion 32 has higher flexibility compared with the first curve portion 31. The third curve portion 33 has the same flexibility compared with the second curve portion 32, but it is also allowed for the third curve portion 33 to have higher flexibility compared with the second curve portion 32.

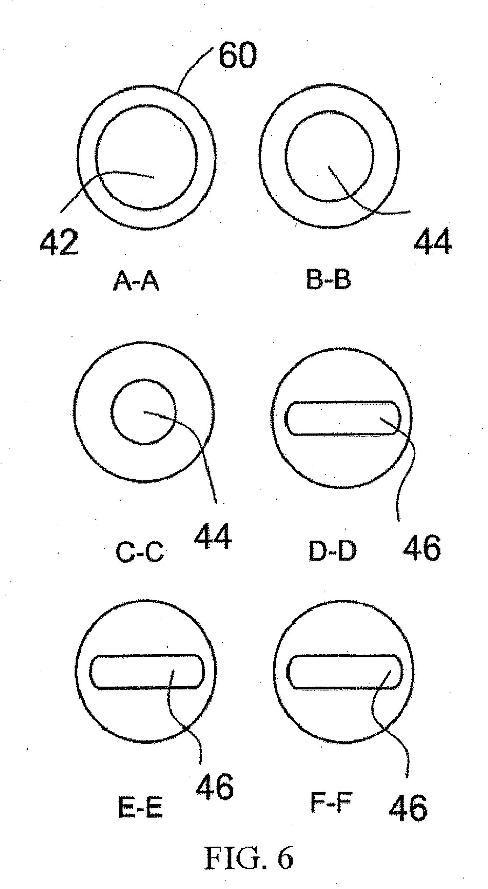

FIG. 6 shows another exemplified embodiment of a guide wire of the present invention and shows end views of respective regions at the positions shown by FIG. 5. There are shown end faces from A-A to F-F which are perpendicular to the axis of the guide wire 1E in FIG. 5.

The A-A end view shows an end face of the main body portion 20 of the guide wire 1E. In the A-A end view, the guide wire 1E is composed of the core wire main body portion 42 of the core wire 40 and the coating portion 60 covering the periphery thereof in a concentric-circle shape.

The B-B end view is an end view of a portion which is positioned on the proximal side compared with the first curve portion 31 and on the axis line of the main body portion 20. The position of the B-B end view is at the taper portion 44 in the core wire 40. The cross-section area of the taper portion 44 is smaller than the cross-section area of the core wire main body portion 42 in the A-A end view.

The C-C end view is an end view at the vertex of the first curve portion 31. The position of the C-C end view is at the taper portion 44 in the core wire 40. The taper portion 44 in the C-C end view is positioned on the distal side compared with the taper portion 44 in the B-B end view. The cross-section area of the taper portion 44 in the C-C end view is smaller than the cross-section area of the taper portion 44 in the B-B end view. The vertex of the first curve portion 31 is positioned between the start portion and the end portion of the taper portion 44 of the core wire 40. The end portion of the taper portion 44 is positioned on the distal side compared with the vertex of the first curve portion 31. The first curve portion 31 is positioned at the taper portion 44 of the core wire 40. The core wire 40 in the first curve portion 31 is in the taper portion 44, so that the first curve portion 31 has higher flexibility compared with the main body portion 20.

The D-D end view is an end view at the vertex of the second curve portion 32. The position of the D-D end view is at the flat portion 46 in the core wire 40. The core wire 40 in the second curve portion 32 is in the flat portion 46, so that the second curve portion 32 has higher flexibility compared with the first curve portion 31. The cross-section area of the flat portion 46 in the second curve portion 32 is smaller than the cross-section area of the taper portion 44 in the first curve portion 31. The thickness of the flat portion 46 is smaller than the outer diameter of the taper portion 44 in the first curve portion 31. Therefore, the second curve portion 32 has higher flexibility compared with the first curve portion 31. By constituting like this, the guide wire does not erroneously-enter into a side-branch even if the most distal tip 12 of the guide wire IE gets into a side-branch caused by a fact that the flexible second curve portion 32 is bent more before the most distal tip 12 is further inserted and the guide wire advances in the main blood vessel with the second curve portion 32 being set at the forefront.

The E-E end view is an end view at the vertex of the third curve portion 33. The position of the E-E end view is at the flat portion 46 in the core wire 40. The core wire 40 at the third curve portion 33 is in the same flat portion 46 as the second curve portion 32, so that the third curve portion 33 has the same flexibility as that of the second curve portion 32.

The F-F end view is an end view at the most distal tip 12. The position of the F-F end view is at the flat portion 46 in the core wire 40. The core wire 40 at the most distal tip 12 is in the same flat portion 46 as the second curve portion 32 and the third curve portion 33, so that the third curve portion 33 has the same flexibility as those of the second curve portion 32 and the third curve portion 33.

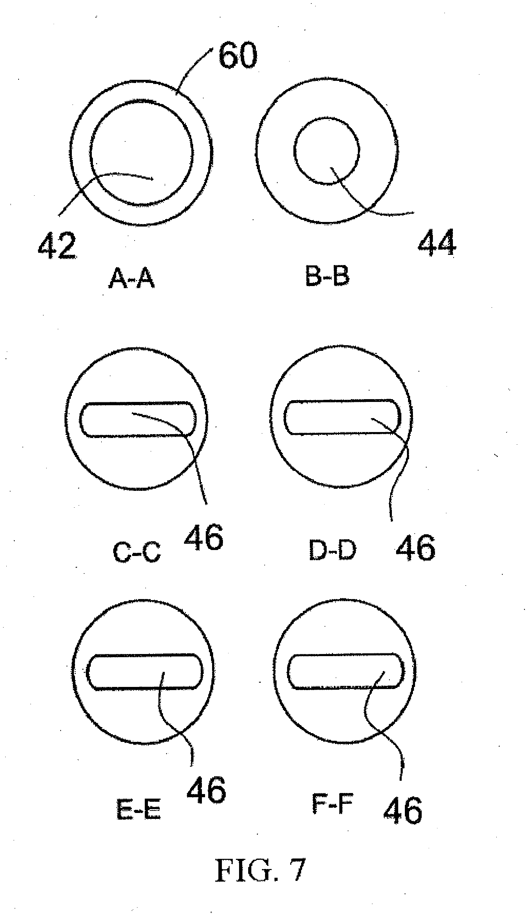

FIG. 7 is an end view showing another exemplified embodiment at the positions shown in FIG. 5. The A-A end view, the B-B end view, the D-D end view, the E-E end view and the F-F end view of the exemplified embodiment shown in FIG. 7 are same as those of FIG. 6, but the core wire 40 in the C-C end view is positioned at the flat portion 46. The cross-section area of the flat portion 46 shown in the C-C end view is smaller than the cross-section area of the taper portion 44 in the B-B end view. The core wire 40 in the first curve portion 31 is in the flat portion 46, so that the first curve portion 31 has higher flexibility compared with the main body portion 20.

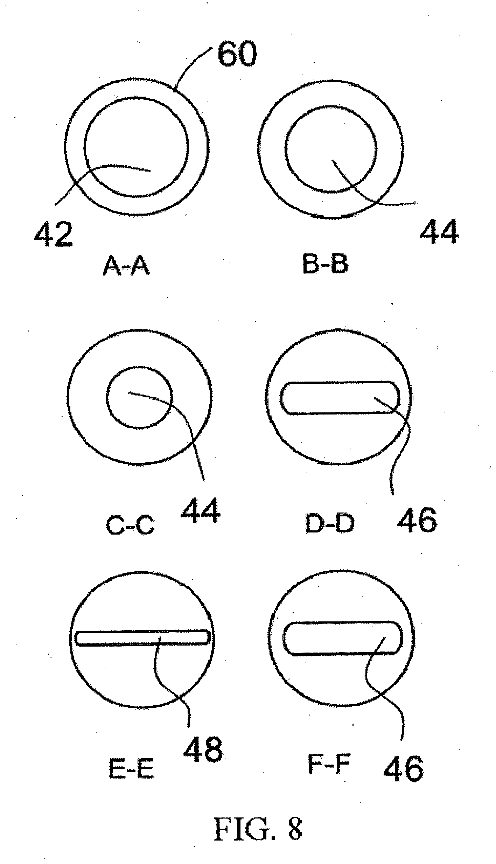

FIG. 8 is an end view showing another exemplified embodiment at the positions shown in FIG. 5. The A-A end view, the B-B end view, the C-C end view, the D-D end view and the F-F end view of the exemplified embodiment shown in FIG. 8 are same as those of FIG. 6, but the E-E end view shows the elastic portion 48 of the core wire 40. As shown in FIG. 4, the elastic portion 48 is made to be thinner in thickness compared with the flat portion 46. The width of the elastic portion 48 is constituted to be wider than the width of the flat portion 46. Consequently, it is constituted such that the third curve portion 33 has higher flexibility compared with the second curve portion 32.

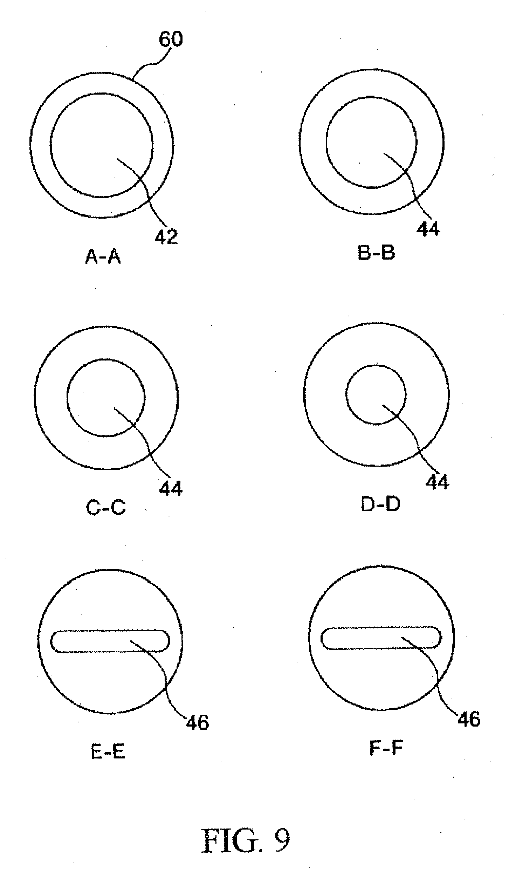

FIG. 9 is an end view showing another exemplified embodiment at the positions shown in FIG. 5. The A-A end view and the B-B end view of the exemplified embodiment shown in FIG. 9 are same as those of FIG. 6. It is constituted such that the cross-section area of the taper portion 44 shown in the C-C end view is larger than the cross-section area of the taper portion 44 shown in the C-C end view in FIG. 6. Also, it is constituted such that the cross-section area of the taper portion 44 shown in the D-D end view is smaller than the cross-section area of the taper portion 44 shown in the C-C end view in the same drawing. Based on such a constitution, the second curve portion 32 has higher flexibility compared with the first curve portion 31. The core wire 40 in the F-F end view and the E-E end view is in the flat portion 46. It is constituted such that the thicknesses of the flat portions 46 in these F-F end view and E-E end view are mutually the same and are smaller than the thickness of the flat portion 46 in the E-E end view (F-F end view) in FIG. 6. Based on such a constitution, the third curve portion 33 will have higher flexibility compared with the first curve portion 31 and the second curve portion 32. Thus, even if the most distal tip 12 of the guide wire gets into a branch, it turns around at the third curve portion 33 and advances in the main blood vessel, and erroneous-entering into a branch is prevented.

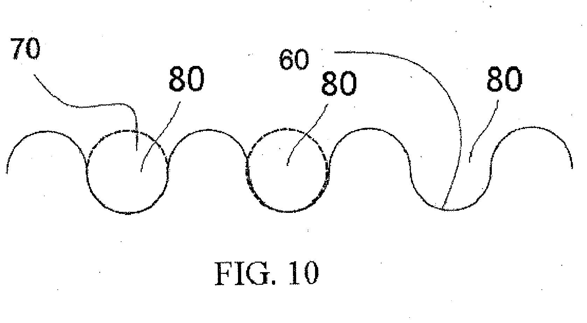

FIG. 10 is a cross-section view showing another exemplified embodiment of a guide wire of the present invention.

FIG. 10 shows an illustrative embodiment for softening the curve portion. In FIG. 10, there is included a groove 80 in a direction almost perpendicular to the axis on the front face of the coating portion 60 of at least the inner side of the curve portion. The groove 80 is provided in a spiral shape. The groove 80 is provided on the front face of the curve portion. For example, the groove 80 is provided at the entire circumference of the front face of the coating portion 60 of the second curve portion 32 and the third curve portion 33 in FIG. 1, FIG. 2 or FIG. 5. The groove 80 is provided only on the front face of the coating portion 60 of the curve portion. As shown in FIG. 10, it is constituted with respect to the horizontal cross-section of the groove 80 such that the vertical cross-section shape of the outer front face (outer circumference face) of the coating portion 60 becomes in a wave shape. It is possible to obtain the wave shaped groove 80, for example, by winding the wire 70 on the coating portion 60 while keeping a space for the outer diameter of the wire 70 and by heating it.

It is also allowed for the groove 80 to be in a loop shape other than in a spiral shape. It is also allowed to employ a slit shape instead of a groove 80. It is also allowed for the groove 80 to be provided only on the front face of the coating portion 60 on the inner side of the curve portion, for example, the second curve portion 32 and the third curve portion 33 in FIG. 1, FIG. 2 or FIG. 5.

In the present exemplified embodiment, flexibility of the curve portion is increased by providing a groove 80 or a slit almost perpendicular to the axis direction on the front face of the coating portion 60 at least on the inner side of the curve portion, so that the curve portion can be bent easily.

Aforesaid illustrative embodiment for softening the curve portion can be used for the exemplified embodiments so far explained.

The illustrative embodiment for softening the curve portion can be also realized by thermally-treating the core wire at the curve portion and by converting it to a material having higher flexibility compared with other portions.

It is possible to use the guide wire of the present invention in order to introduce a medical device of a catheter, a sheath or the like from a radial, a brachial and a femoral to an aimed region of a chest region, an abdominal region or the like and the scope is never limited by the stick region or by the aimed region.

### [Inventive Example]

Next, it will be explained with respect to a concrete inventive example of the present invention.

### 1. Manufacturing of Guide Wire

### (Inventive Example)

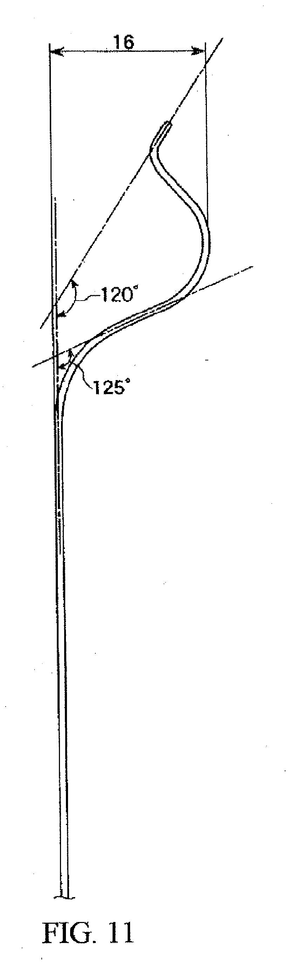

A guide wire shown in FIG. 11 was manufactured. It was constituted such that this guide wire has an outer diameter of 0.89mm and a full length of 1500mm. Also, it was constituted such that the core wire composing the guide wire was made by Ni-Ti and the coating portion was made by polyurethan. Also, with respect to the flat portion, the length thereof was set to be 4mm, the width thereof was set to be 0.23mm and the thickness thereof was set to be 0.03mm. Also, the distance D was about 8.5mm and the distance E was about 13.5mm.

### (Comparative Example 1)

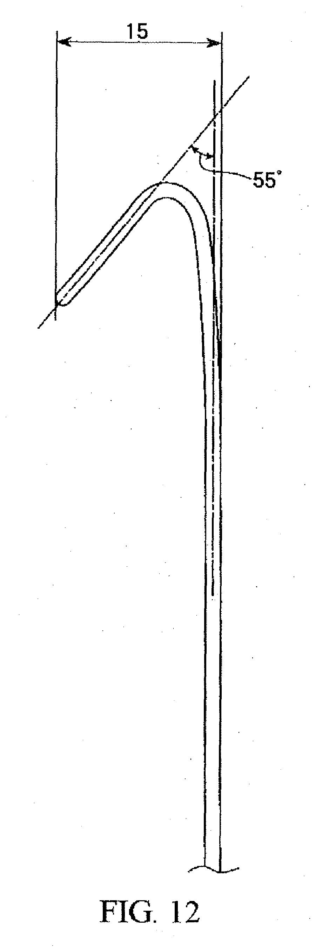

A guide wire shown in FIG. 12 was manufactured. With respect to the outer diameter, the full length and the constituent material of the guide wire; similar specifications as aforesaid inventive example were employed.

### (Comparative Example 2)

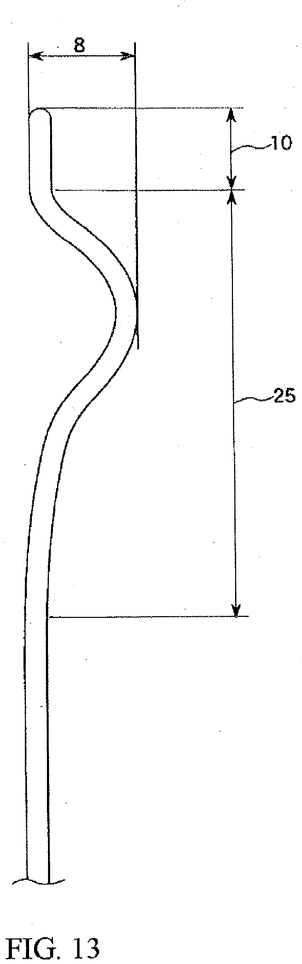

A guide wire shown in FIG. 13 was manufactured. With respect to the outer diameter, the full length and the constituent material of the guide wire; similar specifications as aforesaid inventive example were employed.

### (Comparative Example 3)

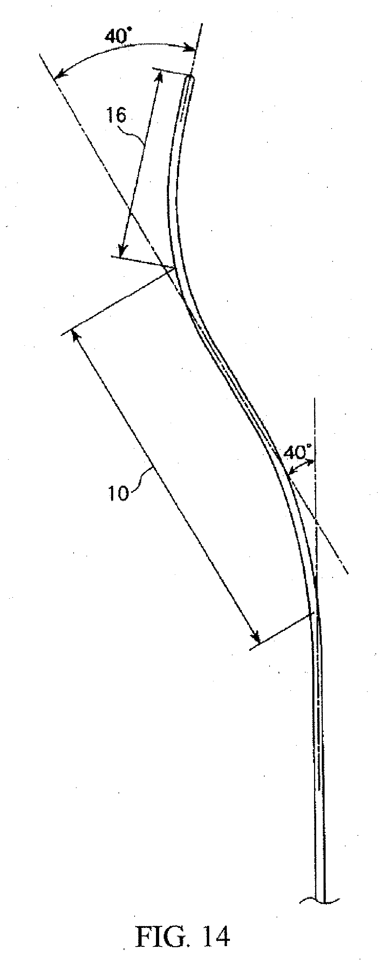

A guide wire shown in FIG. 14 was manufactured. With respect to the outer diameter, the full length and the constituent material of the guide wire; similar specifications as aforesaid inventive example were employed.

### (Comparative Example 4)

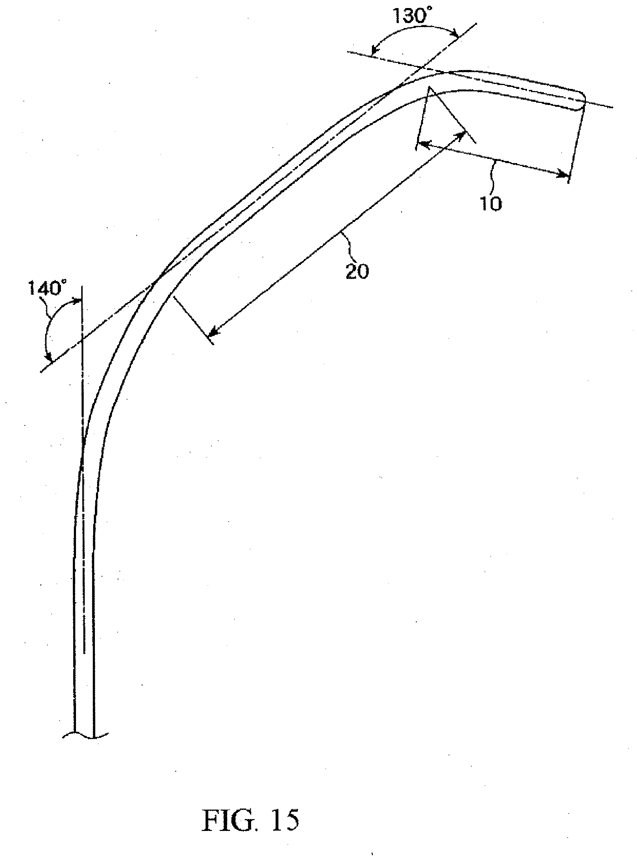

A guide wire shown in FIG. 15 was manufactured. With respect to the outer diameter, the full length and the constituent material of the guide wire; similar specifications as aforesaid inventive example were employed.

### (Comparative Example 5)

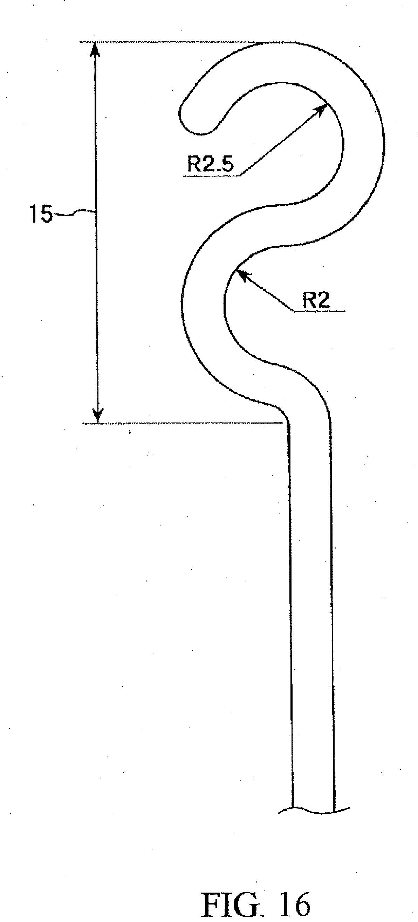

A guide wire shown in FIG. 16 was manufactured. With respect to the outer diameter, the full length and the constituent material of the guide wire; similar specifications as aforesaid inventive example were employed.

It should be noted in FIG. 11 to FIG. 16 that the unit of the length therein is always "mm".

### 2. Evaluation

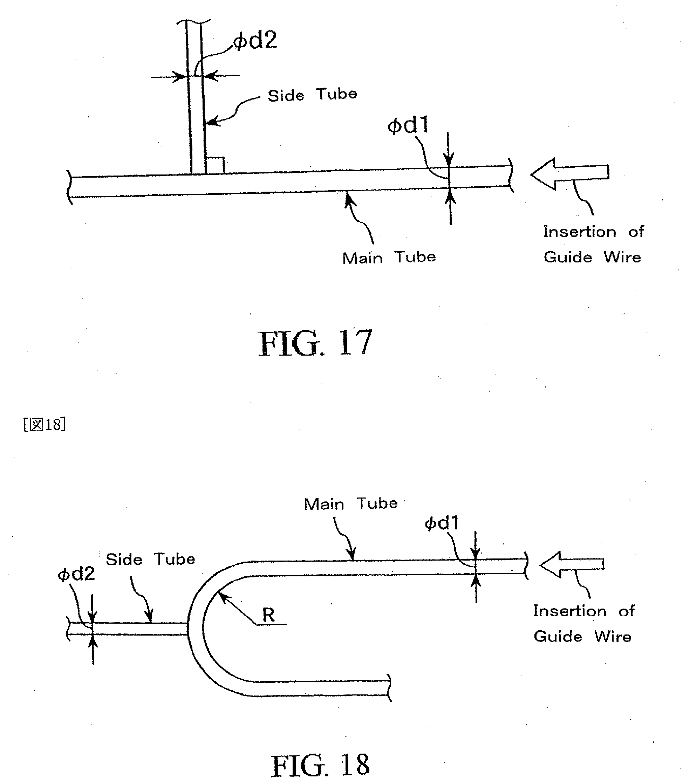

### 2.1 Evaluation by Using Evaluation Tool Shown in FIG. 17

With respect to the guide wires obtained by the inventive example and the respective comparative examples, evaluations were executed respectively by using an evaluation tool shown in FIG. 17. It should be noted that this evaluation tool is a tool in an assumption of a straight-line shaped blood vessel and a side-branch branched on the way of the blood vessel thereof, in which "main tube" corresponds to "straight-line shaped blood vessel" and "side tube" corresponds to "side-branch". Also, it was constituted such that the main tube and the side tube are made by polypropylen respectively.

Here, the guide wire was inserted from the distal side thereof into the main tube five times and evaluation was executed with respect to whether or not the distal portion of the guide wire intrudes into the side tube at least once within those five times. The evaluation criteria were made to be as follows. It should be noted that the evaluation mentioned above was executed by preparing evaluation tools 1A to 5a in which the inner diameters *Φ* d1 of the main tubes and the inner diameters *Φ*d2 of the side tubes are respectively different.

○: means a case in which the distal portion of the guide wire did not intrude into the side tube at all
×: means a case in which the distal portion of the guide wire intruded into the side tube at least once

The evaluation result is shown in table 1.

**Table 1**

| | Evaluation Tool 1a | Evaluation Tool 2a | Evaluation Tool 3a | Evaluation Tool 4a | Evaluation Tool 5a |
|---|---|---|---|---|---|
| | *Φ* d1 : 4mm | *Φ* d1 : 4mm | *Φ* d1 : 5mm | *Φ* d1 : 6mm | *Φ* d1 : 8mm |
| | *Φ* d2 : 1mm | *Φ* d2 : 2mm | *Φ* d2 : 2mm | *Φ* d2 : 3mm | *Φ* d2 : 8mm |
| Inventive Example 1 | ○ | ○ | ○ | ○ | ○ |
| Comparative Example 1 | ○ | ○ | ○ | × | × |
| Comparative Example 2 | ○ | × | ○ | ○ | ○ |
| Comparative Example 3 | ○ | × | × | × | ○ |
| Comparative Example 4 | ○ | × | × | × | × |
| Comparative Example 5 | ○ | ○ | ○ | ○ | ○ |

### 2.2 Evaluation by Using Evaluation Tool Shown in FIG. 18

With respect to the guide wires obtained by the inventive example and the respective comparative examples, evaluations were executed respectively by using an evaluation tool shown in FIG. 18. It should be noted that this evaluation tool is a tool in an assumption of a curved blood vessel (Ulnar Loop) and a side-branch branched from the central portion of the curve portion of the blood vessel thereof toward the outside, in which "main tube" corresponds to "straight-line shaped blood vessel" and "side tube" corresponds to "side-branch". Also, it was constituted such that the main tube and the side tube are made by polypropylen respectively.

Here, evaluation similar as mentioned above was executed by preparing evaluation tools 1b to 5b in which the inner diameters *Φ* d1 of the main tubes and the inner diameters *Φ* d2 of the side tubes are respectively different.

The evaluation result is shown in table 2.

**Table 2**

| | Evaluation Tool 1a | Evaluation Tool 2a | Evaluation Tool 3a | Evaluation Tool 4a | Evaluation Tool 5a |
|---|---|---|---|---|---|
| | *Φ* d1 : 4mm | *Φ* d1 : 4mm | *Φ* d1 : 5mm | *Φ* d1 : 6mm | *Φ* d1 : 8mm |
| | *Φ* d2 : 1mm | *Φ* d2 : 2mm | *Φ* d2 : 2mm | *Φ* d2 : 3mm | *Φ* d2 : 8mm |
| | R : 10mm | R : 10mm | R : 10mm | R : 10mm | R : 20mm |
| Inventive Example 1 | ○ | ○ | ○ | ○ | ○ |
| Comparative Example 1 | ○ | ○ | ○ | ○ | × |
| Comparative Example 2 | ○ | × | ○ | ○ | × |
| Comparative Example 3 | ○ | × | × | × | × |
| Comparative Example 4 | ○ | ○ | ○ | ○ | × |
| Comparative Example 5 | ○ | ○ | ○ | ○ | × |

As clear from table 1 and table 2, with respect to the guide wire obtained by the inventive example, the distal portion of the guide wire did not intrude into the side tube at all even in a case in which the evaluation is executed by any one of the evaluation tools.

On the other hand, with respect to the guide wires obtained by respective comparative examples, there occurred a case in which the distal portion of the guide wire intruded into the side tube at least once in any one of the evaluation tools.

Also, the guide wires shown in FIG. 2 and FIG. 5 to FIG. 10 were manufactured and evaluations similar to that of aforesaid inventive example were executed also with respect to them. Consequently, similar results as that of aforesaid inventive example could be obtained also with respect to the guide wires shown in the respective drawings.

### 2.3 Evaluation by Using Catheter

With respect to the guide wires obtained by the inventive example and the respective comparative examples, evaluations were executed respectively by using a catheter. It should be noted that this catheter is a catheter including a tube-shaped catheter main body having elasticity and a hub installed at the proximal portion of aforesaid catheter main body. Also, the hub is a hub having a cylindrical shape and communicates with the catheter main body. Also, the inner diameter of the catheter main body was *Φ* 1.05mm and the inner diameter of the hub entrance port was *Φ* 4mm.

Here, insertion of the guide wire from the distal side thereof with respect to the hub was attempted five times and evaluation was executed with respect to whether or not the distal portion of the guide wire was not inserted into the hub at least once within those five times. The evaluation criteria were made to be as follows.

○: means a case in which the distal portion of the guide wire could be inserted all five times
× : means a case in which the distal portion of the guide wire could not be inserted at least once

The evaluation result is shown in table 3.

**Table 3**

| | Catheter |
|---|---|
| | Inner Diameter of Catheter Main Body: *Φ* 1.05 |
| | Inner Diameter of Hub Input Port: *Φ* 4 |
| Inventive Example 1 | ○ |
| Comparative Example 1 | ○ |
| Comparative Example 2 | ○ |
| Comparative Example 3 | ○ |
| Comparative Example 4 | ○ |
| Comparative Example 5 | × |

As clear from table 3, with respect to the guide wire obtained by the inventive example, the distal portion of the guide wire could be inserted into the hub all five times.

On the other hand, with respect to the guide wires obtained by respective comparative examples, there occurred a case in which the distal portion of the guide wire could not be inserted into the hub at least once.

Also, the guide wires shown in FIG. 2 and FIG. 5 to FIG. 10 were manufactured and evaluations similar to that of aforesaid inventive example were executed also with respect to them. Consequently, similar results as that of aforesaid inventive example could be obtained also with respect to the guide wires shown in the respective drawings.

### INDUSTRIAL APPLICABILITY

The guide wire of the present invention is a guide wire which includes a distal portion and a main body portion and which is provided with a first curve portion; a second curve portion included on the distal side of aforesaid first curve portion and curved to the opposite direction with respect to aforesaid first curve portion; a third curve portion included on the distal side of aforesaid second curve portion and curved to the opposite direction with respect to aforesaid second curve portion, wherein a line contacting with both aforesaid first curve portion and aforesaid third curve portion has an obtuse angle with respect to an axis line of aforesaid main body portion. For this reason, it is excellent in steerability when the guide wire is operated. Therefore, the guide wire of the present invention includes industrial applicability.

## Claims

1. A guide wire (1A - IE) comprising a distal portion (10), having a most distal tip (12), and a main body portion (20) wherein:
said distal portion (10) of the guide wire includes a first curve portion (31);
a second curve portion (32) included on the distal side of said first curve portion (31) and curved to the opposite direction with respect to said first curve portion;
a third curve portion (33) included on the distal side of said second curve portion (32) and curved to the opposite direction with respect to said second curve (32) portion, wherein the first curve portion (31), the second curve portion (32) and the third curve portion (33) are constituted in the same plane;
wherein in a state in which no external force is applied to the guide wire (1A - IE), a straight line (A) contacting with both said first curve portion (31) and said third curve portion (33) without intersecting the guide wire body, has, an obtuse angle (180°-K) with respect to an axis line (Y) of said main body portion (20),
wherein said first curve portion (31) has higher flexibility compared with said main body portion (20) and said second curve portion (32) has higher flexibility compared with said first curve portion (31),
wherein the direction (M) of the portion (71) shifting from said first curve portion (31) to said second curve portion (32) has a larger angle (K) with respect to said axis line (Y) compared with the direction (N) which said most distal tip (12) is directed with respect thereto.

## Patentansprüche

1. Führungsdraht (1A - 1E), umfassend einen distalen Abschnitt (10), der eine am weitesten distal gelegene Spitze (12) aufweist, und einen Hauptkörperabschnitt (20),
wobei:
der distale Abschnitt (10) des Führungsdrahts einen ersten Krümmungsabschnitt (31) aufweist;
einen zweiten Krümmungsabschnitt (32), der auf der distalen Seite des ersten Krümmungsabschnitts (31) enthalten ist und in die entgegengesetzte Richtung in Bezug auf den ersten Krümmungsabschnitt gekrümmt ist;
einen dritten Krümmungsabschnitt (33), der auf der distalen Seite des zweiten Krümmungsabschnitts (32) eingeschlossen ist und in die entgegengesetzte Richtung in Bezug auf den zweiten Krümmungsabschnitt (32) gekrümmt ist, wobei der erste Krümmungsabschnitt (31), der zweite Krümmungsabschnitt (32) und der dritte Krümmungsabschnitt (33) in derselben Ebene gebildet sind;
wobei in einem Zustand, in dem keine äußere Kraft auf den Führungsdraht (1A - 1E) ausgeübt wird, eine gerade Linie (A), die sowohl den ersten Krümmungsabschnitt (31) als auch den dritten Krümmungsabschnitt (33) berührt, ohne den Führungsdrahtkörper zu schneiden, einen stumpfen Winkel (180°-K) in Bezug auf eine Achsenlinie (Y) des Hauptkörperabschnitts (20) aufweist,
wobei der erste Krümmungsabschnitt (31) im Vergleich zum Hauptkörperabschnitt (20) eine höhere Flexibilität aufweist und der zweite Krümmungsabschnitt (32) im Vergleich zum ersten Krümmungsabschnitt (31) eine höhere Flexibilität aufweist,
wobei die Richtung (M) des Abschnitts (71), die sich von dem ersten Krümmungsabschnitt (31) zu dem zweiten Krümmungsabschnitt (32) verschiebt, einen größeren Winkel (K) in Bezug auf die Achsenlinie (Y) aufweist, im Vergleich mit der Richtung (N), in welche die am weitesten distal gelegene Spitze (12) in Bezug auf diese gerichtet ist.

## Revendications

1. Fil guide (1A - 1E) comprenant une partie distale (10), ayant une pointe distale extrême (12), et une partie corps principal (20) où :
ladite partie distale (10) du fil guide comprend une première partie courbe (31) ;
une seconde partie courbe (32) incluse sur le côté distal de ladite première partie courbe (31) et courbée vers la direction opposée par rapport à ladite première partie courbe ;
une troisième partie courbe (33) incluse sur le côté distal de ladite seconde partie courbe (32) et courbée vers la direction opposée par rapport à ladite seconde partie courbe (32), où la première partie courbe (31), la seconde partie courbe (32) et la troisième partie courbe (33) sont constituées dans le même plan ;
où
dans un état où aucune force externe n'est appliquée au fil guide (1A - 1E), une ligne droite (A) contactant à la fois ladite première partie courbe (31) et ladite troisième partie courbe (33) sans couper le corps de fil guide, a un angle obtus (180°-K) par rapport à un axe (Y) de ladite partie corps principal (20),
où ladite première partie courbe (31) a une plus grande flexibilité comparée à celle de ladite partie corps principal (20) et ladite seconde partie courbe (32) a une plus grande flexibilité comparée à celle de ladite première partie courbe (31),
où la direction (M) de la partie (71) allant de ladite première partie courbe (31) à ladite seconde partie courbe (32) a un plus grand angle (K) par rapport audit axe (Y) par comparaison avec la direction (N) vers laquelle ladite pointe distale extrême (12) est dirigée.
